# EUROPEAN PATENT APPLICATION

(11) **EP 3 769 761 A1**
(43) Date of publication of application: **27.01.2021**
(21) Application number: 19188597.9
(22) Date of filing: 26.07.2019
(51) Int. Cl.: A61K 31/395, A61P 9/00

(54) **CXCR4 ANTAGONISTS FOR TREATMENT OF CARDIOVASCULAR CONDITIONS**

(71) Applicant: Charité - Universitätsmedizin Berlin, 10117 Berlin (DE)
(72) Inventor: Kararigas, Georgios, 13086 Berlin (DE)
(74) Representative: Winter, Brandl, Fürniss, Hübner, Röss, Kaiser, Polte - Partnerschaft mbB

(57) **Abstract**

The present invention relates to a CXCR4 antagonist for use in the treatment of a patient suffering from a cardiovascular condition, a pharmaceutical composition comprising a CXCR4 antagonist for use in the treatment of a patient suffering from a cardiovascular condition, a method of diagnosis of a patient suffering from a cardiovascular condition based on a determination of CXCR4 expression in a patient, and a kit comprising a CXCR4 antagonist for use in the treatment of a patient suffering from a cardiovascular condition.

SEQUENCE LISTING

<110> Charite - Universitaetsmedizin Berlin

<120> CXCR4 antagonists for treatment of cardiovascular conditions

<130> CB1901P-EP

<160> 4

<170> BiSSAP 1.3.6

<210> 1
<211> 20
<212> DNA
<213> Artificial Sequence


<220>
<223> HPRT1 forward primer

<400> 1
ctttgctgac ctgctggatt                                                20


<210> 2
<211> 20
<212> DNA
<213> Artificial Sequence


<220>
<223> HPRT1 reverse primer

<400> 2
tatgtcccct gttgactggt                                                20


<210> 3
<211> 20
<212> DNA
<213> Artificial Sequence


<220>
<223> CXCR4 forward primer

<400> 3
ggtggtctat gttggcgtct                                                20


<210> 4
<211> 20
<212> DNA
<213> Artificial Sequence


<220>
<223> CXCR4 reverse primer

<400> 4
ctcactgacg ttggcaaaga                                                20

## Description

### Technical field

The present invention relates to a CXCR4 antagonist for use in the treatment of a patient suffering from a cardiovascular condition, a pharmaceutical composition comprising a CXCR4 antagonist for use in the treatment of a patient suffering from a cardiovascular condition, a method of diagnosis of a patient suffering from a cardiovascular condition based on a determination of CXCR4 expression in a patient, and a kit comprising a CXCR4 antagonist for use in the treatment of a patient suffering from a cardiovascular condition.

### Background of the Invention

Cardiovascular (CV) diseases are the number one cause of death, globally representing 31.5% of all deaths. In Europe, cardiovascular diseases are responsible for over 4 million deaths per year (Nichols M, et al. Cardiovascular disease in europe: Epidemiological update. Eur Heart J. 2013;34:3028-3034).

Heart failure (HF), a deficiency in the ability of the heart to pump sufficient amounts of blood in response to systemic demands, is a cardiovascular disorder with increasing incidence and prevalence that could be most appropriately considered an epidemic of enormous socioeconomic consequences. Heart failure is a devastating syndrome with poor prognosis that is most often preceded by a left ventricular (LV) pressure overload (PO) disorder.

Despite considerable advances in pharmacological, surgical and technology-based cardiovascular therapy, the morbidity and mortality of those afflicted with heart failure remain high. Notably, there are significant differences in the development and outcome of heart failure between different patients, with male-female differences being prominent (Martinez-Selles M, et al. Gender and survival in patients with heart failure: Interactions with diabetes and aetiology. Results from the maggic individual patient meta-analysis. Eur J Heart Fail. 2012;14:473-479), but mechanisms are poorly understood.

Pressure overload due to aortic stenosis (AS) or hypertension constitutes a major burden for the heart and as pressure inside the left ventricle increases, left ventricular hypertrophy (LVH) develops. Under left ventricular hypertrophy, persistent inflammation is known to be detrimental to the heart, and the inflammatory response might promote heart failure (Vasan RS, et al. Framingham Heart S. Inflammatory markers and risk of heart failure in elderly subjects without prior myocardial infarction: The framingham heart study. Circulation. 2003;107:1486-1491).

In fact, activation of the immune response and pro-inflammatory factors lead to myocardial fibrosis and the inhibition of cardiac contractility. Impaired contractile function of the heart is a major risk factor for heart failure and sudden death. Elevated circulating levels of pro-inflammatory cytokines, such as tumour necrosis factor (TNF)-α and interleukin (IL)-6, play a pivotal role in HF (Bozkurt B, et al. Biomarkers of inflammation in heart failure. Heart Fail Rev. 2010;15:331-341). Cytokine production is also increased in the failing myocardium (Torre-Amione G, et al. Proinflammatory cytokine levels in patients with depressed left ventricular ejection fraction: A report from the studies of left ventricular dysfunction (SOLVD). J Am Coll Cardiol. 1996;27:1201-1206, and Wrigley BJ, et al. The role of monocytes and inflammation in the pathophysiology of heart failure. Eur J Heart Fail. 2011;13:1161-1171).

Cardiac cells releasing inflammatory mediators, such as the cytokine chemokine (C-X-C motif) ligand 12 (CXCL12) [synonym: stromal cell-derived factor-1 (SDF-1)], promote a pro-inflammatory environment, as chemokines and their receptors are crucial for leukocyte and fibrocyte migration. An overall hypothesis may be that increased recruitment of pro-inflammatory cells and fibrocytes expressing chemokine receptors, such as the CXCL12 receptor CXCR4 [chemokine (C-X-C motif) receptor 4], might be critical for progression of cardiovascular diseases, as it contributes significantly to the development of inflammation and myocardial fibrosis.

In cardiovascular disease, most experimental efforts have focused on understanding immune mechanisms in response to ischaemic heart injury provoked by myocardial infarction and not in pressure overload.

Importantly, there are significant differences in the development and outcome of left ventricular diseases between men and women (Regitz-Zagrosek V and Kararigas G. Mechanistic pathways of sex differences in cardiovascular disease. Physiol Rev. 2017;97:1-37). In particular, women develop more often concentric hypertrophy with smaller ventricular diameter, greater relative wall thickness and a better systolic function, while men demonstrate more pronounced chamber dilation and systolic dysfunction.

As a result, in heart failure, women have a better survival rate than men. However, the mechanisms that cause these sex differences are poorly understood. To this extent, it may be hypothesised that male-specific increased recruitment of pro-inflammatory cells after injury may be critical for progression of disease.

Furthermore, in progressive heart failure, elevated sympathetic tone ultimately leads to desensitization of the sympathetic response. Therefore, normalisation of the cardiac sympathetic input has been an intense area of investigation. However, attempts at modulating sympathetic tone with drugs, such as phosphodiesterase inhibitors and chronic inotropes, have not been successful. More importantly, a major therapeutic obstacle is that drugs designed to enhance cardiac contraction promote arrhythmias. Therefore, the identification of new therapeutic targets is of utmost importance.

Aortic stenosis causes pressure overload and leads to left ventricular hypertrophy that progresses towards heart failure and sudden death. The treatment of aortic stenosis depends on its stage - early and/or mild forms are monitored, often under propanolol treatment, however from a certain critical stage onward surgical aortic valve replacement (AVR) is the standard intervention and currently also the only available treatment. Aortic stenosis is the most common adult heart valve disease in western countries and its prevalence is rising with increasing life expectancy. Aortic valve replacement is expected to result in the regression of left ventricular hypertrophy and improvement of cardiac function.

Nevertheless, this does not occur in all patients and even among the patients undergoing left ventricular hypertrophy regression, the pattern may be distinct between different individuals. In fact, studies hint at a more favourable regression pattern in women (Petrov G, et al. Regression of myocardial hypertrophy after aortic valve replacement: Faster in women? Circulation. 2010;122:S23-28, and Villar AV, et al. Myocardial gene expression of microrna-133a and myosin heavy and light chains, in conjunction with clinical parameters, predict regression of left ventricular hypertrophy after valve replacement in patients with aortic stenosis. Heart. 2011;97:1132-1137).

However, the role of sex in left ventricular hypertrophy regression is currently under debate, since some studies report no major impact or even the opposite, depending on the interpretation of the data. Thus, the prior art does not allow a reliable conclusion regarding the role of sex in this context. Persistence of left ventricular hypertrophy following aortic valve replacement however imposes a negative prognosis for these patients by increasing the risk for congestive heart failure, coronary artery disease and sudden death. Effective pharmacological treatments to improve LVH regression are currently not available.

The onset of the regression of LVH may already occur early after AVR (Djavidani B, et al. Early regression of left ventricular hypertrophy after aortic valve replacement by the ross procedure detected by cine mri. J Cardiovasc Magn Reson. 2004;6:1-8). Since regression of LVH is associated with better long-term outcome, it is of major clinical relevance to identify existing and novel interventions relevant for clinical success.

Previously, faster regression of LVH and better survival after AVR was observed in older women compared to men (Petrov G, et al. Maladaptive remodeling is associated with impaired survival in women but not in men after aortic valve replacement. JACC Cardiovasc Imaging. 2014;7:1073-1080). Investigating the underlying mechanisms, it was found that maladaptive remodelling, which occurs more frequently in men, is associated with greater activation of inflammatory transcriptional profiles (Kararigas G, et al. Sex-dependent regulation of fibrosis and inflammation in human left ventricular remodelling under pressure overload. Eur JHeart Fail. 2014;16:1160-1167).

Notably, previous studies employing the mouse under pressure overload conditions applying the transverse aortic constriction (TAC) method are in agreement with findings in humans, showing, among others, sex-specific regulation of inflammatory genes and pathways (Kararigas G, et al. Comparative proteomic analysis reveals sex and estrogen receptor beta effects in the pressure overloaded heart. J Proteome Res. 2014;13:5829-5836, Kararigas G, et al. Role of the estrogen/estrogen-receptor-beta axis in the genomic response to pressure overload-induced hypertrophy. Physiol Genomics. 2011;43:438-446, and Fliegner D, et al. Female sex and estrogen receptor-beta attenuate cardiac remodeling and apoptosis in pressure overload. Am J Physiol Regul Integr Comp Physiol. 2010;298:R1597-1606).

These findings led to the overall hypothesis that a persistent inflammatory response worsens left ventricular hypertrophy and hinders its regression, which may occur in a sex-specific manner. The role of sex in this context, however, cannot be reliably determined. As discussed above, the state of the art also suffers from problems in the treatment of cardiovascular diseases in that normalisation of the cardiac sympathetic input and enhanced cardiac contraction could not successfully be achieved without the risk of significant side effects.

Furthermore, it is not understood why certain patients suffering from cardiovascular diseases such as left ventricular hypertrophy caused by aortic stenosis profit significantly from aortic valve replacement and show regression of said hypertrophy and why this is not the case for other patients. Effective pharmacological treatments to improve LVH regression are currently not available.

Thus, it is an object of the present invention to provide a novel target to achieve enhanced cardiac contraction in patients suffering from cardiovascular conditions, such as left ventricular hypertrophy, and treatment options to improve cardiovascular diseases such as left ventricular hypertrophy regression, in particular following aortic valve replacement, without the risk of significant side effects such as arrhythmias.

### Summary of the Invention

These objects have been solved by the aspects of the present invention as specified hereinafter.

According to the first aspect of the present invention, a CXCR4 antagonist is provided for use in the treatment of a patient suffering from a cardiovascular condition.

According to a preferred embodiment of the first aspect of the present invention, the cardiovascular condition is any one of left ventricular pressure overload (LV-PO), aortic stenosis (AS), aortic valve replacement (AVR), hypertension, left ventricular hypertrophy (LVH), persistent inflammation of the heart, systemic inflammatory response syndrome (SIRS), maladaptive remodelling of the heart, increased LV mass, decreased ejection fraction and/or heart disease with increased inflammation, more preferably wherein maladaptive remodelling of the heart is defined by one or more of increased left ventricular end-diastolic diameter (LVEDD), increased left ventricular mass and decreased relative wall thickness (RWT), even more preferably by a positive value of the LV remodelling index.

According to another preferred embodiment of the first aspect of the present invention, the cardiovascular condition is characterized by one or more of the following parameters being fulfilled in the patient: fever or hypothermia with a temperature of less than 36.0°C or of more than 38.0°C, tachycardia with a heart rate of more than 90 beats per minute, hyperventilation characterized by a respiratory rate of more than 20 breaths per minute or a PaCO₂ of less than 32 mmHg, leucocytosis or leucopenia characterized by a leucocyte count of more than 12 × 10⁹/L or less than 4 × 10⁹/L, increased inflammatory biomarkers including interleukin-6 (IL-6) of more than 15 pg/mL, interleukin-8 (IL-8) of more than15 pg/mL, and/or C-reactive protein (CRP) of more than 10 mg/L.

According to one preferred embodiment of the first aspect of the present invention, the cardiovascular condition of the patient to be treated is left ventricular hypertrophy (LVH) and the patient to be treated has been subject to aortic valve replacement (AVR), more preferably the patient to be treated has been subject to aortic valve replacement (AVR) within the last five years.

According to yet another preferred embodiment of the first aspect of the present invention, the cardiovascular condition is not ischemia-induced.

According to a preferred embodiment of the first aspect of the present invention, the CXCR4 antagonist is POL6326 (balixafortide), BMS-936564 (MDX-1338), BKT140 (4F-benzoyl-TN14003), or AMD3100 (plerixafor), preferably the CXCR4 antagonist is AMD3100 (plerixafor).

According to another preferred embodiment of the first aspect of the present invention, the patient does not suffer from HIV infection, WHIM syndrome, solid malignant tumors, multiple myeloma and/or non-Hodgkin lymphoma.

According to one preferred embodiment of the first aspect of the present invention, the CXCR4 antagonist is not co-administered together with the granulocyte-colony stimulating factor (G-CSF).

According to a preferred embodiment of the first aspect of the present invention, the patient is male.

According to one other preferred embodiment of the first aspect of the present invention, the patient has a level of CXCR4 expression in relation to normalised CXCR4 levels of the general population of more than 1.0 fold of the normalised CXCR4 level of the general population, more preferably more than 1.5 fold, even more preferably more than 2.0 fold.

According to one preferred embodiment of the first aspect of the present invention, the patient has been found to be eligible for treatment with the CXCR4 antagonist prior to treatment.

According to a second aspect of the present invention, a method of diagnosis of a patient suffering from a cardiovascular condition is provided, wherein a level of CXCR4 expression is determined in relation to the normalised CXCR4 level of the general population, and wherein the patient is determined to be eligible for treatment with a CXCR4 antagonist if the level of CXCR4 expression is more than 1.0 fold of the normalised CXCR4 level of the general population, more preferably more than 1.5 fold, even more preferably more than 2.0 fold of the normalised CXCR4 level of the general population.

According to a third aspect of the present invention, a pharmaceutical composition is provided comprising a CXCR4 antagonist according to the first aspect, and at least one pharmaceutically acceptable excipient for use in the treatment of a patient suffering from a cardiovascular condition.

According to a preferred embodiment of the third aspect of the present invention, the pharmaceutical composition does not comprise granulocyte-colony stimulating factor (G-CSF).

According to another preferred embodiment of the third aspect of the present invention, the pharmaceutical composition is administered to the patient by way of systemic administration, more preferably wherein the pharmaceutical composition is administered to the patient by way of oral administration, intravenous administration or subcutaneous administration, even more preferably by oral administration or subcutaneous administration, specifically preferably by oral administration.

According to one preferred embodiment of the third aspect of the present invention, the pharmaceutical composition comprises the antagonist in a range of from 0.02 to 0.40 mg/kg body weight, more preferably in a range of 0.05 to 0.30 mg/kg body weight, even more preferably in a range of 0.10 to 0.28 mg/kg body weight, particularly preferably in a range of 0.2 to 0.25 mg/kg body weight.

According to a preferred embodiment of the third aspect of the present invention, the pharmaceutical composition is administered between twice daily and once every two days, more preferably the pharmaceutical composition is administered daily.

According to a fourth aspect of the present invention, a kit is provided comprising a CXCR4 antagonist according to the first aspect of the present invention, and a container for use in the treatment of a patient suffering from a cardiovascular condition.

### Description of Figures

Figure 1 shows relative CXCR4 expression in LVH samples of male (n=9) and female (n=10) patients in arbitrary units in comparison.
Figure 2 shows the effect of administration of AMD3100 as CXCR4 antagonist to mice subjected to TAC (transverse aortic constriction) regarding the heartweight to tibia ratio (HW/TL) in male mice (n=4 in each group).
Figure 3 shows the effect of administration of AMD3100 as CXCR4 antagonist to mice subjected to TAC (transverse aortic constriction) regarding the ejection fraction (in %) in male mice (n=4 in each group).

### Detailed Description of the Invention

The present inventor has dedicated himself to solving the problem of the present invention and were successful to find a novel molecular target and novel methods which are suitable to improve cardiovascular diseases without the risk of significant side effects such as arrhythmias.

Based on the recognition that persistent inflammatory response worsens left ventricular hypertrophy and hinders its regression, potentially in a sex-specific manner, proteins involved therein have been analysed in depth. In particular, CXCR4 gene levels have been characterised in cardiomyocytes isolated from left ventricular samples of patients suffering from a cardiovascular disease. Furthermore, administration of a CXCR4 antagonist to TAC mice (subjected to transverse aortic constriction or TAC) mimicking aortic stenosis revealed positive effects to the development of heart structure and function.

While CXCR4 antagonists have previously been used in the clinical environment, CXCR4 antagonism with the small organic bicyclam molecule plerixafor (AMD3100) is normally employed to mobilise and harvest haematopoietic progenitor cells for bone marrow transplants. Experimental studies have also shown that CXCR4 inhibition via plerixafor results in neutrophil mobilization from reservoirs other than the bone marrow.

Applications of CXCR4 antagonists in the cardiovascular field appear to be related mostly to events involving or following ischemia or infarction due to its effects in mobilization of progenitor cells, with the expectation that this would enhance regeneration of the heart. However, these known applications appear unrelated to the persistent inflammatory setting in progression towards heart failure.

On the basis of novel and surprising data regarding the use of CXCR4 antagonists in cardiovascular disease, activation of CXCR4 associated with increased pro-inflammatory cell infiltration is assumed to lead to impaired cardiac function. Consequently, pharmacological inhibition of CXCR4 with a specific antagonist such as plerixafor may confer patients having a relevant level of CXCR4 expression with protection from certain cardiovascular conditions including pressure overload-induced left ventricular hypertrophy.

Furthermore, it is put forward that CXCR4 antagonists such as plerixafor (or any other relevant inhibitor) will facilitate the regression of LVH and myocardial function following aortic valve replacement.

The data presented herein suggest a new role for CXCR4 in cardiovascular disease such as pressure overload-induced left ventricular hypertrophy. Consequently, another novel aspect of the present invention is that CXCR4 antagonism would confer aortic stenosis patients undergoing aortic valve replacement functional protection, which could be a new indication for plerixafor, with a direct impact on guidelines. This may emerge as an important intervention common to the recovery of wide-range cardiovascular events that contribute to cardiovascular disease epidemics, thereby having a major impact on patient prognosis and life quality, as well as savings to health care systems.

Thus, the present invention provides a CXCR4 antagonist for use in the treatment of a patient suffering from a cardiovascular condition.

Within the present invention, the cardiovascular condition may be any condition of a patient which is positively affected by administration of a CXCR4 antagonist. Preferably, the cardiovascular condition according to the present invention is any one of left ventricular pressure overload (LV-PO), aortic stenosis (AS), aortic valve replacement (AVR), hypertension, left ventricular hypertrophy (LVH), persistent inflammation of the heart, systemic inflammatory response syndrome (SIRS), maladaptive remodelling of the heart, increased LV mass, decreased ejection fraction and/or heart disease with increased inflammation.

In the context of the present invention, it is to be understood that patients suffering from the cardiovascular condition "aortic valve replacement" shall encompass all patients having had an aortic (heart) valve replacement carried out. According to a preferred embodiment of the present invention, the cardiovascular condition of the patient to be treated is left ventricular hypertrophy, wherein the patient to be treated has previously been subject to aortic valve replacement.

According to a particularly preferable embodiment of the present invention, the CXCR4 antagonist is for use in facilitating the regression of left ventricular hypertrophy and/or myocardial function following an aortic valve replacement in a patient.

According to another preferable embodiment of the present invention, maladaptive remodelling of the heart as the cardiovascular condition is defined by one or more of increased left ventricular end-diastolic diameter (LVEDD), increased left ventricular mass and decreased relative wall thickness (RWT), preferably by a positive value of the LV remodelling index which take the three aforementioned parameters into account.

For a determination of maladaptive remodelling of the heart, preferably in case of chronic pressure overload, LV end-diastolic diameter (LVEDD) can be used as a marker for dilation, and LV mass and relative wall thickness (RWT) as markers for concentricity. A combination of LV mass, LV end-diastolic diameter, and relative wall thickness may be included in a factor analysis to generate an index classifying LV remodelling as adaptive or maladaptive (cf. Kararigas G, et al. Sex-dependent regulation of fibrosis and inflammation in human left ventricular remodelling under pressure overload. Eur J Heart Fail. 2014;16:1160-1167). Mean and peak transvalvular pressure gradients can also be included.

The measures related to LV remodelling are preferably combined in the LV remodelling index and forced orthogonal to the PO index (combines mean and peak transvalvular pressure gradients). The Kaiser-Meyer-Olkin (KMO) measure of sampling adequacy in the reported publication was found to be 0.374, while the Bartlett's test of sphericity was highly significant (*P* < 0.0001). Principal component analysis with Varimax rotation was used as the underlying factor-analytical method. The Kaiser-Guttman criterion was applied to ascertain the number of extracted factors.

Accordingly, the LV remodelling index can be used to classify echocardiographic LV remodelling into adaptive or maladaptive patterns. Since the LV remodelling index is a z-transformed variable, positive index values correlate with an increase in LVEDD (factor loading 0.962) and LV mass (factor loading 0.661) and a decrease in RWT (factor loading -0.818), thereby reflecting maladaptive LV remodelling. The LV remodelling pattern was considered adaptive when the criteria for maladaptive LV remodelling were not fulfilled, i.e. negative LV remodelling index values.

Thus, according to one preferred embodiment of the present invention, maladaptive remodeling is considered to be present in patients having a positive index value of the LV remodeling index.

According to one other preferred embodiment of the present invention, the cardiovascular condition, in particular persistent inflammation of the heart, systemic inflammatory response syndrome (SIRS) and/or heart disease with increased inflammation, is characterized by one or more, more preferably two or more, even more preferably three or more, of the following parameters being fulfilled in a patient:
- fever or hypothermia (temperature <36.0°C or >38.0°C)
- tachycardia (heart rate >90 beats/min)
- hyperventilation (respiratory rate >20 breaths/min or PaCO₂ <32 mmHg)
- leucocytosis or leucopenia (leucocyte count >12 × 10⁹/L or <4 × 10⁹/L)
- increased inflammatory biomarkers:
   - interleukin-6 (IL-6, >15 pg/mL)
   - interleukin-8 (IL-8, >15 pg/mL)
   - C-reactive protein (CRP, >10 mg/L).

With regard to the list of parameters given above, any value stated therein is to be considered one parameter with the proviso that the two values given for hyperventilation are preferably mutually exclusive. In a more preferable embodiment, four or more of the above parameters are fulfilled, further preferably five or more, even further preferably six or more. In another preferred embodiment, the cardiovascular condition is characterized by at least one parameter of the inflammatory biomarkers IL-6, IL-8 or CRP as defined above being fulfilled.

According to another preferable embodiment of the present invention, the cardiovascular condition of the patient to be treated is left ventricular hypertrophy (LVH) and the patient to be treated has been subject to aortic valve replacement (AVR), more preferably wherein the patient to be treated has been subject to aortic valve replacement (AVR) within the last five years, even more preferably within the last two years, even more preferably within the last year, particularly preferably within the last six months.

According to one preferable embodiment of the present invention, the cardiovascular condition is not ischemia-induced or infarct-induced. According to another embodiment of the present invention, the patient to be treated does not suffer from HIV infection, WHIM syndrome, solid malignant tumors, multiple myeloma and/or non-Hodgkin lymphoma.

According to another preferred embodiment of the present invention, the CXCR4 antagonist is not co-administered together with the granulocyte-colony stimulating factor (G-CSF). Preferably, the CXCR4 antagonist or a pharmaceutical composition comprising the CXCR4 antagonist is administered to the patient in a dosage of the CXCR4 antagonist in a range of from 0.02 to 0.40 mg/kg body weight, preferably in a range of 0.05 to 0.30 mg/kg body weight, more preferably in a range of 0.10 to 0.28 mg/kg body weight, particularly preferably in a range of 0.2 to 0.25 mg/kg body weight of the patient.

According to one preferred embodiment of the present invention, the CXCR4 antagonist or a pharmaceutical composition comprising the CXCR4 antagonist is administered to the patient by way of systemic administration, preferably by way of oral administration, intravenous administration or subcutaneous administration, more preferably by oral administration or subcutaneous administration., even more preferably by oral administration.

Preferably, the CXCR4 antagonist or a pharmaceutical composition comprising the CXCR4 antagonist is administered to the patient between three times daily and twice a week, more preferably between twice daily and once every two days, even more preferably the pharmaceutical composition is administered daily.

In principle, the CXCR4 antagonist of the present invention may be any molecule acting as an antagonist to CXCR4 function. According to a preferable embodiment of the present invention, the CXCR4 antagonist may comprise a molecule from the group consisting of AMD3100 (plerixafor), POL6326 (balixafortide), BL-8040, mavorixafor, burixafor, Q-122, PTX-9908, ulocuplumab, USL-311, GMI-1359, LY-2510924, hz-515H7, HPH-12, KY-1051, POL-5551, BKT-300, AD-214, X-4P002, X-4P003, AM-3114, PF-06747143, X4-136, ALB-408, ALX-0651, HPH-196, HPH-211, CS-3955, BKT-170, STIA-220X, LY-2624587, NB-325, AT-009, SP-10, CTCE-9908, GBV-4086, procaine hydrochloride, BMS-936564 (MDX-1338), BKT140 (4F-benzoyl-TN14003), or any other known CXCR4 antagonist, more preferably from the group consisting of POL6326 (balixafortide), BMS-936564 (MDX-1338), BKT140 (4F-benzoyl-TN14003), or AMD3100 (plerixafor), even more preferably the CXCR4 antagonist is AMD3100 (plerixafor).

According to one preferred embodiment of the present invention, the patient is male. Preferably within the present invention, being male is defined by the patient having as sex chromosomes one X chromosome and one Y chromosome.

According to another preferred embodiment of the present invention, the patient has a level of CXCR4 expression in relation to normalised CXCR4 levels of the general population of more than 1.0 fold, preferably more than 1.5 fold, more preferably more than 2.0 fold. As normalised CXCR4 levels of the general population, the average level of CXCR4 expression of the general population across all sexes is preferably adopted.

Thus, CXCR4 expression levels may preferably be determined by a given method for a representative average group of an equal number of males and females from the general population. The values found by the given determination method should then be added and divided by the number of values to obtain a normalized value for CXCR4 levels of the general population. Said normalized value will then be set as 1.0 fold and compared to the CXCR4 level determined for the patient by the same method.

In case that the value for the patient is higher than the normalised value for CXCR4 levels of the general population, the patient has a level of more than 1.0 fold in relation to normalised CXCR4 levels of the general population. Similar comparisons between the patient's CXCR4 level and the normalized value for the general population may be made according to simple arithmetic operations.

Preferably, the CXCR4 expression levels are determined as follows.

Biopsies are collected from either the interventricular septum or left ventricular lateral wall of patients undergoing cardiac surgery, e.g. aortic valve replacement. Samples are harvested with a surgical scalpel from the left ventricular side of the interventricular septum or with a Tru-Cut needle from the epicardial lateral wall (80 mg per patient).

All samples are immediately snap frozen in liquid nitrogen and stored at -80°C. RNA is isolated with commercially available kits, e.g. the RNeasy Mini Kit (Qiagen), following the manufacturer's instructions. RNA is reverse transcribed and amplified using commercially available kits, e.g. One Step RT qPCR MasterMix (Eurogentec), following the manufacturer's instructions. PCR products are obtained using gene-specific, intron-spanning primers and SYBR Green (Applied Biosystems) in a 7000 ABI Prism Instrument (Applied Biosystems). The levels of *CXCR4* are normalised to hypoxanthine phosphoribosyltransferase 1 (*HPRT1*). Normalisation would also be possible relative to any other generally known housekeeping gene.

Primer sequences are for *HPRT1* forward 5'-FW CTT TGC TGA CCT GCT GGA TT-3' and reverse 5'-TAT GTC CCC TGT TGA CTG GT-3', and for *CXCR4* forward 5'-FW GGT GGT CTA TGT TGG CGT CT-3' and reverse 5'-CTC ACT GAC GTT GGC AAA GA-3'.

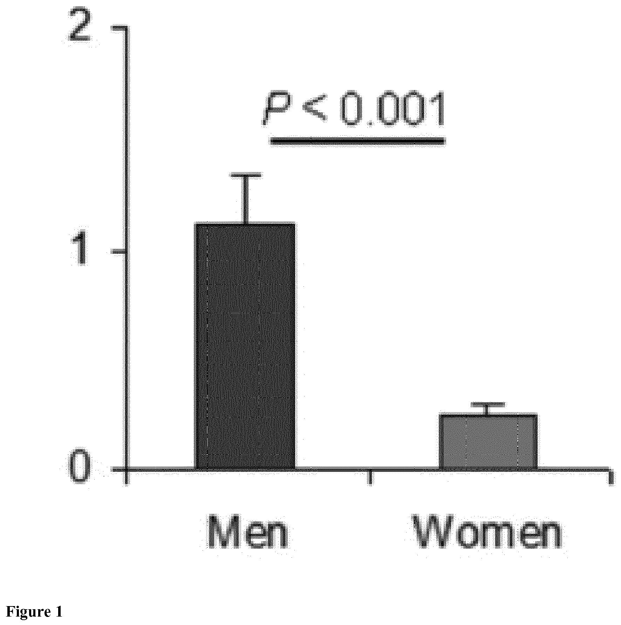

Judging from the experiment shown in Figure 1, "unaffected" female patients included in this study appear to have an average of 0.25 *CXCR4* expression levels expressed in arbitrary units, while male patients demonstrated CXCR4 expression levels in arbitrary units which are four times as high in comparison to the screened females.

Based on this discrepancy between the relative difference between male patients and female patients, it appears possible and feasible to define a cut-off value for CXCR4 expression. Such a cut-off value would be suitable to discriminate between male patients having unusually low CXCR4 levels and would not profit from the treatment with CXCR4, female patients having unusually high CXCR4 expression levels that would profit from the treatment, and other females (not eligible for CXCR4 antagonist treatment) and males (eligible for CXCR4 antagonist treatment) in general.

According to one preferred embodiment, the patient suffering from a cardiovascular condition has been found to be eligible for treatment with the CXCR4 antagonist prior to treatment. Preferably, eligibility for treatment with the CXCR4 antagonist may be based on male sex and/or CXCR4 expression levels.

In one aspect, the present invention is also directed to a method of treatment of a patient suffering from a cardiovascular condition, wherein the patient is administered an effective amount of CXCR4 antagonist according to the invention or a pharmaceutical composition of the invention.

In another aspect, the present invention is also directed to the use of a CXCR4 antagonist of the invention or a pharmaceutical composition of the invention in the manufacture of a medicament for treatment of a cardiovascular condition.

Additionally, the present invention also relates to a method of diagnosis of a patient suffering or suspected to be suffering from a cardiovascular condition wherein a level of CXCR4 expression is determined in relation to normalised CXCR4 levels of the general population, and wherein the patient is determined to be eligible for treatment with a CXCR4 antagonist if the level of CXCR4 expression is more than 1.0 fold, preferably more than 1.5 fold, more preferably more than 2.0 fold of the normalised CXCR4 level of the general population. Preferably, the assay as described above is used for determining the CXCR4 level of the patient.

The present invention further relates to a pharmaceutical composition comprising a CXCR4 antagonist as defined and further described herein and at least one pharmaceutically acceptable excipient for use in the treatment of a patient suffering from a cardiovascular condition.

Preferably, the pharmaceutical composition may further comprise antibodies, biologicals, synthetic protein epitope mimetic peptidic antagonists, synthetic peptides, inhibitory nucleic acids (receptor and ligand) encompassing siRNA, miRNA, shRNA, aptamers and spiegelmers, decoy antibody variants, and others.

In accordance with the above, the pharmaceutical composition preferably does not comprise granulocyte-colony stimulating factor (G-CSF).

Further preferably, the pharmaceutical composition of the present invention is administered to the patient by way of systemic administration, preferably the pharmaceutical composition is administered to the patient by way of oral administration, intravenous administration or subcutaneous administration, more preferably by oral administration or subcutaneous administration, even more preferably by oral administration.

According to a preferred embodiment of the present invention, the pharmaceutical composition comprises the CXCR4 antagonist in a range of from 0.02 to 0.40 mg/kg body weight, preferably in a range of 0.05 to 0.30 mg/kg body weight, more preferably in a range of 0.10 to 0.28 mg/kg body weight, particularly preferably in a range of 0.2 to 0.25 mg/kg body weight of the patient.

The present invention also encompasses a kit comprising a CXCR4 antagonist as defined herein, or a pharmaceutical composition as defined herein, and a container for use in the treatment of a patient suffering from a cardiovascular condition.

In conclusion, the present invention found that certain cardiovascular diseases have a CXCR4 dependent pathology which may be positively influenced by the administration of CXCR4 antagonists to patients in need thereof.

All embodiments of the present invention as described herein are deemed to be combinable in any combination, unless the skilled person considers such a combination to not make any technical sense.

### Examples

### 1. CXCR4 gene levels

RNA was isolated with the RNeasy Mini Kit (Qiagen) following the manufacturer's instructions. RNA was reverse transcribed and amplified using One Step RT qPCR MasterMix (Eurogentec) following the manufacturer's instructions. PCR products were obtained using gene-specific, intron-spanning primers and SYBR Green (Applied Biosystems) in a 7000 ABI Prism Instrument (Applied Biosystems).

The levels of CXCR4 were normalized to hypoxanthine phosphoribosyltransferase 1 (HPRT1). Primer sequences are for HPRT1 forward 5'-FW CTT TGC TGA CCT GCT GGA TT-3' and reverse 5'-TAT GTC CCC TGT TGA CTG GT-3', and for CXCR4 forward 5'-FW GGT GGT CTA TGT TGG CGT CT-3' and reverse 5'-CTC ACT GAC GTT GGC AAA GA-3'.

CXCR4 gene levels were characterized in cardiomyocytes isolated from left ventricular samples of male (n = 9) and female (n = 10) aortic stenosis patients undergoing aortic valve replacement, showing higher expression in male patients in comparison to female patients. The values shown in Figure 1 are given in arbitrary units and demonstrate that women have an average value of 0.25 arbitrary units, while men have an average value of more than 1.0 arbitrary units. In spite of the small sample, the significant difference between the average values for men and women is striking and clearly indicative of a significant difference in CXCR4 expression in men and women.

The results of this determination of relative CXCR4 levels is shown in Figure 1.

### 2. Transthoracic echocardiography analysis of TAC mice treated with a CXCR4 antagonist

Two-month-old mice were anesthetized with isoflurane (4%) in oxygen. After hemi-thoracotomy, the transverse aorta was ligated with a 5-0 silk suture (FST) against a 26-gauge needle to carry out transverse aortic constriction (TAC). Sham animals underwent an identical surgical procedure without placement of a suture. Animals recovered from anesthesia under warming conditions and normal ventilation.

The treatment with AMD3100 (plerixafor) as the CXCR4 antagonist (5 mg/kg daily subcutaneous injection) was started 4 days after TAC. Cardiac function and geometry were assessed by transthoracic echocardiography (Vevo 2100) in anesthetised mice (2.5% isoflurane) with body temperature maintained at 37°C. LV internal dimensions at end-diastole (LVIDd) and end-systole (LVIDs), and LV posterior wall (LVPW) were measured from M-mode images.

LV ejection fraction was calculated from the two-dimensional parasternal long-axis view using the single-plane area-length method. LV mass was calculated using the equation: LV mass = 1.055[(IVST + LVEDD + PWT)3 - (LVEDD)3], where 1.055 is the specific gravity of the myocardium, IVST is the interventricular septal thickness and PWT is the posterior wall thickness. After 31 days, the mice were sacrificed by isoflurane overdose followed by laryngotomy.

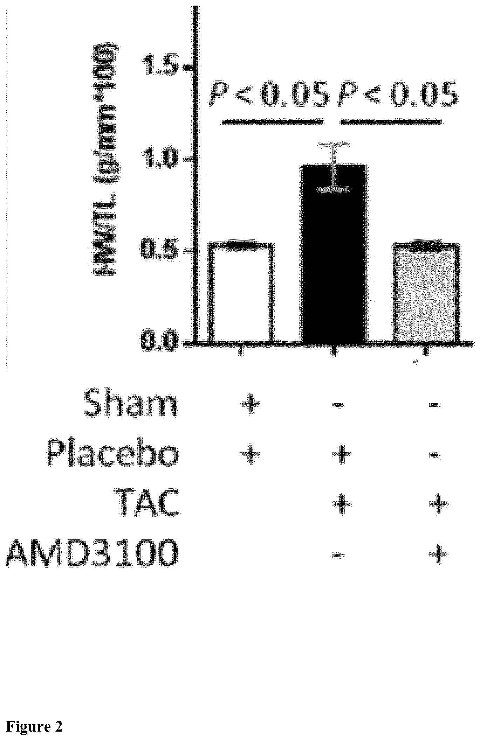

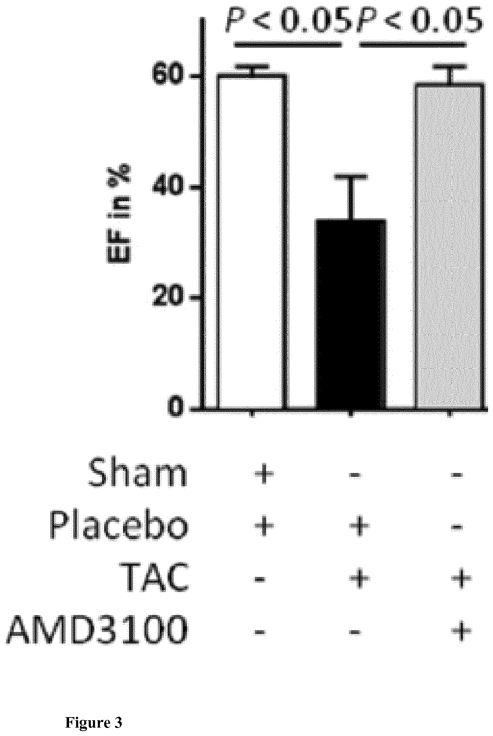

Results obtained from these experiments are shown in Figure 2 (heart-weight-to-tibia-length ratio (HW/TL) in (g/mm)*100) and Figure 3 (ejection fraction) for sham mice treated with placebo, TAC mice treated with placebo and TAC mice with the CXCR4 antagonist AMD3100 (plerixafor).

## Claims

1. CXCR4 antagonist for use in the treatment of a patient suffering from a cardiovascular condition.

2. CXCR4 antagonist for use according to claim 1, wherein the cardiovascular condition is any one of left ventricular pressure overload (LV-PO), aortic stenosis (AS), aortic valve replacement (AVR), hypertension, left ventricular hypertrophy (LVH), persistent inflammation of the heart, systemic inflammatory response syndrome (SIRS), maladaptive remodelling of the heart, increased LV mass, decreased ejection fraction and/or heart disease with increased inflammation, preferably wherein maladaptive remodelling of the heart is defined by one or more of increased left ventricular end-diastolic diameter (LVEDD), increased left ventricular mass and decreased relative wall thickness (RWT), preferably by a positive value of the LV remodelling index.

3. CXCR4 antagonist for use according to claim 1 or 2, wherein the cardiovascular condition is **characterized by** one or more, more preferably two or more, even more preferably three or more, of the following parameters being fulfilled in the patient:
- fever or hypothermia (temperature <36.0°C or >38.0°C)
- tachycardia (heart rate >90 beats/min)
- hyperventilation (respiratory rate >20 breaths/min or PaCO₂ <32 mmHg)
- leucocytosis or leucopenia (leucocyte count >12 × 10⁹/L or <4 × 10⁹/L)
- increased inflammatory biomarkers:
- interleukin-6 (IL-6, >15 pg/mL)
- interleukin-8 (IL-8, >15 pg/mL)
- C-reactive protein (CRP, >10 mg/L).

4. CXCR4 antagonist for use according to any of claims 1 to 3, wherein the cardiovascular condition of the patient to be treated is left ventricular hypertrophy (LVH) and the patient to be treated has been subject to aortic valve replacement (AVR), preferably wherein the patient to be treated has been subject to aortic valve replacement (AVR) within the last five years.

5. CXCR4 antagonist for use according to any of claims 1 to 4, wherein the cardiovascular condition is not ischemia-induced and/or wherein the patient does not suffer from HIV infection, WHIM syndrome, solid malignant tumors, multiple myeloma and/or non-Hodgkin lymphoma.

6. CXCR4 antagonist for use according to any one of claims 1 to 5, wherein the CXCR4 antagonist is POL6326 (balixafortide), BMS-936564 (MDX-1338), BKT140 (4F-benzoyl-TN14003), or AMD3100 (plerixafor), preferably wherein the CXCR4 antagonist is AMD3100 (plerixafor), and/or wherein the CXCR4 antagonist is not co-administered together with the granulocyte-colony stimulating factor (G-CSF).

7. CXCR4 antagonist for use according to any one of claims 1 to 6, wherein the patient is male.

8. CXCR4 antagonist for use according to any one of claims 1 to 7, wherein the patient has a level of CXCR4 expression in relation to normalised CXCR4 levels of the general population of more than 1.0 fold, preferably more than 1.5 fold, more preferably more than 2.0 fold.

9. CXCR4 antagonist for use according to any one of claims 1 to 10, wherein the patient has been found to be eligible for treatment with the CXCR4 antagonist prior to treatment.

10. Method of diagnosis of a patient suffering or suspected to be suffering from a cardiovascular condition, wherein a level of CXCR4 expression is determined in relation to the normalised CXCR4 level of the general population, and wherein the patient is determined to be eligible for treatment with a CXCR4 antagonist if the level of CXCR4 expression is more than 1.0 fold, preferably more than 1.5 fold, more preferably more than 2.0 fold of the normalised CXCR4 level of the general population.

11. Pharmaceutical composition comprising a CXCR4 antagonist according to any one of claims 1 to 9 and at least one pharmaceutically acceptable excipient for use in the treatment of a patient suffering from a cardiovascular condition.

12. Pharmaceutical composition for use according to claim 11, wherein the pharmaceutical composition is administered to the patient by way of systemic administration, preferably wherein the pharmaceutical composition is administered to the patient by way of oral administration, intravenous administration or subcutaneous administration, more preferably by oral administration or subcutaneous administration, even more preferably by oral administration.

13. Pharmaceutical composition for use according to claim 11 or 12, wherein the pharmaceutical composition comprises the antagonist in a range of from 0.02 to 0.40 mg/kg body weight, preferably in a range of 0.05 to 0.30 mg/kg body weight, more preferably in a range of 0.10 to 0.28 mg/kg body weight, particularly preferably in a range of 0.2 to 0.25 mg/kg body weight, and/or wherein the pharmaceutical composition does not comprise granulocyte-colony stimulating factor (G-CSF).

14. Pharmaceutical composition for use according to any of claims 11 to 13, wherein the pharmaceutical composition is administered between twice daily and once every two days, preferably wherein the pharmaceutical composition is administered daily.

15. Kit comprising a CXCR4 antagonist according to any one of claims 1 to 9 and a container for use in the treatment of a patient suffering from a cardiovascular condition.
